(19) Europäisches Patentamt
European Patent Office
Office européen des brevets

(11) **EP 2 208 531 A1**

(12) **EUROPEAN PATENT APPLICATION**

(43) Date of publication:
**21.07.2010 Bulletin 2010/29**

(51) Int Cl.:
*B01L 3/00* (2006.01)    *G01N 33/543* (2006.01)
*G01N 35/00* (2006.01)    *G01N 1/40* (2006.01)

(21) Application number: **08173113.5**

(22) Date of filing: **30.12.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Atonomics A/S**
**2450 Copenhagen SV (DK)**

(72) Inventors:
• **Warthoe, Peter**
  **2100 Copenhagen Ø (DK)**

• **Mentzel, Søren**
  **2300 Copenhagen S (DK)**
• **Mikkelsen, Jens**
  **2750 Ballerup (DK)**
• **Sørensen, Iben Schildt**
  **2200 Copenhagen N (DK)**

(74) Representative: **Christensen, Bent et al**
  **Chas. Hude A/S**
  **H.C. Andersens Boulevard 33**
  **1780 Copenhagen V (DK)**

(54) **Distribution of particles in capillary channel by application of magnetic field**

(57)    The present invention relates to a device for the quantitative detection of the presence or absence of a target analyte in a liquid sample, and to uses thereof. The invention further relates to a method for quantitative detecting the presence or absence of a target analyte in a sample consisting of less than 200 µl. Further the invention relates a system and a method for clustering or homogenously distributing a plurality of paramagnetic particles in a capillary channel. The system comprises a channel device and a magnetic device. The homogenously distribution was obtained by moving an external magnetic field of the magnetic device with field lines substantially perpendicular to the longitudinal direction of the channel. Thereby the accuracy and precision of a given assay was improved, especially for small quantities of analyte; background unspecific signal was reduced or eliminated; and the signal arising from the analyte was increased.

Fig. 8

**Description**

Technical Field

The present invention relates to a device for separating a suspension into a liquid phase and a retentate phase and to the use thereof.

The invention further relates to a method for separating a liquid sample consisting of less than 200 µl suspension, into a retentate phase comprising the suspended matter, and a liquid phase substantially free of suspended matter. The suspension might be blood, the liquid phase plasma/serum and the retentate blood cells.

The invention further relates to a device for quantitative detecting the presence or absence of one or more target analyte(s) in a liquid sample, and to uses thereof.

The invention further relates to a method for quantitative detecting the presence or absence of one or more target analyte(s) in a sample consisting of less than 200 µl.

The invention further relates a system for clustering and/or homogenously distributing a plurality of paramagnetic particles in a capillary channel. The system comprises a channel device and a magnetic device.

Background

Key advantages of using microfluidic systems of reduced dimension for analytical applications are known to be (I) the possibility of using minute quantities of sample and reagents (down to picoliters), (II) relatively fast reaction times, when molecular diffusion lengths are of the order of the microchannel dimension, and (III) a large surface-to-volume ratio offering an intrinsic compatibility between the use of a microfluidic system and surface-based assays.

Nano and microparticles have become a hot topic in research. Functional nano- and microparticles ('beads') offer a large specific surface for chemical binding and a polymer colloid or microsphere solution has a low viscosity compared to solutions having the same amount of solid, giving it special properties. Such small particles can be advantageously used as a 'mobile substrate' for bio-assays.

The most prominent advantage of magnetic beads over other solid phases lies in the fact that the particles can be magnetically probed and manipulated using permanent magnets or electromagnets, independent of normal chemical or biological processes. Some of the classical applications, like magnetic separation, have already found their way down to miniaturized fluidic or 'lab-on-a-chip' systems that strongly limit the consumption of samples and reagents; in such systems, magnetic beads effectively provided a chemically active substrate with a large surface-to-volume ratio. The booming area of miniaturized applications of magnetic beads offers many exciting possibilities for future developments. It is a highly multidisciplinary area, requiring contributions from inorganic chemistry involved in the preparation of the magnetic beads, through biochemistry and medical science to allow for their functionalization and, of course, the basic physics of magnetism and magnetic materials. Nearly all important functions in a bio-assay can be realized using magnetic beads: sample purification, providing a solid substrate to the sample, mixing, labelling, manipulation and transport and, finally, separation. Combining such particles and their detection system with the recently developed magnetic manipulation techniques already resulted in highly integrated bio-assay systems, in which all functions (from sample treatment to read-out) could be extremely miniaturized.

Magnetic nano- and microparticles offer still an additional advantage: having embedded magnetic entities, they can be magnetically manipulated using permanent magnets or electromagnets, independent of normal microfluidic or biological processes. This extra degree of freedom is at the basis of a still improved exposure of the functionalized bead surface to the surrounding liquid and of higher sample pre-concentration efficiencies, due to the increased relative motion of the bead with respect to the fluid.

**Different types of Magnetic Beads**

In early publications, magnetic bead solutions were produced by grinding magnetite ($Fe_3O_4$) with long-chain hydrocarbons and a grinding agent. Later magnetic fluids were produced by precipitating an aqueous $Fe^{3+}/Fe^{2+}$ solution with a base, coating these particles with an adsorbed layer of oleic acid and then dispersing them in a non-aqueous fluid. Both type of processes result in very small magnetic particles with a surfactant coating in a non-aqueous liquid carrier in which the hydrophobic magnetite particles are dispersed.

However, a lot of applications of magnetic beads rely on water as the continuous phase. A water-based magnetic fluid was realized by conversion of iron products to magnetic iron oxide in an aqueous medium under controlled pH conditions. Polymer-coated magnetic beads can be produced by in situ precipitation of magnetic materials in the presence of a polymer. In this way, magnetic beads surrounded by a hydrophilic polymer shell have been made, choosing for the polymer the water-soluble dextran, poly(ethyleneimine), poly(vinyl alcohol), poly(ethylene glycol) etc.

Different types of magnetic beads have their individual advantages and disadvantages. Polystyrene-coated magnetic particles are known for their excellent size distribution and spherical shape. However, their hydrophobic surface results in a high amount of unspecific protein and antibody binding on the particle surface, so that it needs to be modified chemically. Magnetic silica particles are very efficient in adsorbing proteins and DNA on their surface, but are hardly available with a small size distribution and an ideal spherical shape. Magnetic polysaccharide particles are important for many in vivo applications.

They combine biocompatibility with availability in a size range below 300 nm, but the particles are irregular in shape and the soft particle matrix causes them to be sensitive to mechanical stress. Also magnetic poly (lactic acid) particles play an important role in in vivo applications they are biodegradable and their degradation time in the blood can be adjusted by their molecular weight and exact chemical composition.

From a physics point of view, the magnetic content of a particle is of particular importance. Small nanoparticles consist of a single-domain, because they have a dimension that is typically of the order or smaller than the typical thickness of a magnetic domain wall.

Mono-domain magnetic particles become super paramagnetic (i.e. their time averaged magnetization without a magnetic field is zero), when their magnetic energy is lower than about 10 times the thermal energy $K_B$ T , with r the particle radius of a supposed spherical particle and KB the Boltzmann constant. At room temperature, $K_B$ T $= 4.0 \times 10^{-21}$ J, and one finds a maximum radius r = 6 nm for a super paramagnetic spherical particle of iron. Iron oxides, such as magnetite ($Fe_3O_4$) or maghemite ($\gamma$-$Fe_2O_3$) are more stable against oxidation and are preferentially used as core material, instead of iron. Typically, super paramagnetic particles of $Fe_3O_4$ with diameters in the 5-100 nm range are used. The magnetization curve of an ensemble of such super paramagnetic particles is hysteresis-free. As a consequence, suspended super paramagnetic particles tagged to the biomaterial of interest can be removed from a matrix using a magnetic field, but they do not agglomerate after removal of the field (i.e. they stay suspended). Hence, it is very easy to switch-on and -off the magnetic interaction. Larger magnetic particles (typically 0.5-5 $\mu$m in diameter) can have a single magnetic core or have a core composed of multiple more or less magnetically interacting nanoparticles in a non-magnetic matrix.

Such mostly have a multi-domain structure and are characterized by a hysteretic magnetization characteristic. Two of such particles will have an maximum magnetic attraction energy much larger than the thermal energy, resulting in strong dipolar forces between the particles. As a consequence, when exposed to an external magnetic induction, the magnetic microparticles acquire a magnetic dipole moment and coalesce, under influence of the magnetic dipole interaction into a supra-particle structure (SPS) consisting of chain-like 'columnar structures along the field direction.

**Forces on Magnetic Beads**

**Subtitle: Why use a permanent magnet and not an electro magnet?**

It is important to recognize that a magnetic field gradient is required to exert a translation force, a uniform field giving rise only to a torque. The magnetic force (F) acting on a point-like magnetic dipole or magnetic moment (m) can be written as the gradient of the magnetic energy (Zborowski et al. Journal of Magnetism and Magnetic Materials 194 (1-3):224-230).

$$F = \frac{1}{\mu} V(mB) \approx \frac{1}{\mu} (mV)B$$

The second part of the equation holds when the magnetic moment of the particles is not varying in space (V m) = 0.

However this is only correct assumption when the moment is permanent or the magnetic particles is in such large field that its magnetization is completely saturated. In the case of superparamagnetic particles in a liqied medium, one can write for the moment m = V $\mu M$ = V$\mu XH$, with M the magnetization, V the volume of the particle and X the difference in magnetic susceptibility between the particle and the medium. Using the relation B = $\mu$ H, following F can be obtained:

$$F = \frac{VX}{\mu} (BV)B$$

A permanent magnet typically is characterized by a magnetic induction Bm = 0.5 - 1 T and a field gradient VB $\approx$ Bm /w, with w the geometrical dimension of the permanent magnet. For a cylindrical permanent magnet with a diameter $\varnothing$ = 5 mm, one induces on a spherical particle with radius r = 500 nm and X = 1 a magnetic moment m = $2.6 \times 10^{-19}$ T$m^3$, resulting in a magnetic force of about 40 pN. For a current-fed coil, the generated field is much smaller: a flat millimeter-size coil with 10 windings and a current of 0.5 - 1 A generates typically a magnetic induction of 1-10 mT, at least 100 times smaller than the permanent magnet. Consequently, also the gradient is a factor 100 lower, so that the force (F) is a factor $10^4$ larger, explaining the frequent use of permanent magnets for actuation of magnetic beads.

In many applications, a magnetically labelled material is separated from a liquid solution by passing the fluid mixture through a region where there is a magnetic field gradient that can immobilize the tagged material via magnetic forces. In all examples, the magnetic force needs to be appreciably larger than the hydrodynamic drag force acting on the magnetic particle.

The hydrodynamic drag force $F_d$ is a consequence of the velocity difference between the magnetic particle and the liquid $\Delta V$ and, for a spherical particle with radius r, is given by:

$$F_d = 6 \, \pi \, \eta \, r \, \Delta v \, f_D$$

Where $\eta$ is the viscosity of the medium surrounding the particle (for water, $\eta = 8.9 \times 10^{-4}$ N smE-2) and $f_D$ the drag coefficient that takes into account the influence of an eventual solid wall in the vicinity of the moving particle. Far away from the wall, $fD \approx 1$, while, when approaching the wall to a distance that is comparable with the particle size, $f_D$ can increase up to a factor 3.

Besides the magnetic and viscous forces that act in the horizontal plane of the cartridges, there exist other types of forces that are aligned perpendicularly to the channel wall, like the electrostatic force and the Van der Waals force. The Van der Waals force between two atoms is in most cases an attractive force that originates from electromagnetic interactions between dipoles and/or induced dipoles and that is acting on a distance smaller than 0.1 - 1 $\mu$m.

When a substrate or a particle is immersed into an aqueous solution, a surface charge can be induced through adsorption of ions present in the liquid and/or dissociation of surface groups. This surface charge is neutralized by oppositely charged mobile charges in the liquid, forming the so called double layer. When the double layer of two surfaces overlap, an electrostatic interaction is induced resulting in either a repulsive or an attractive force.

Another important force is gravity. For reference, assuming a bead has a mass density of 10 times water, a bead with a diameter of 1 $\mu$m will experience a gravitational force 51 fN: the magnetic force on the bead is ten times greater than that from gravity. The values used for this calculation are fairly conservative. One could clearly increase the expected force on magnetizable particles by several orders of magnitude by increasing the external field and the particles' susceptibility and volume.

**Magnetic Bead Separation**

Magnetic separator design can be as simple as the application and removal of a permanent magnet to the wall of a test tube to cause aggregation, followed by removal of the supernatant. However, it is preferable to increase the separator efficiency by producing regions with a high magnetic field gradient to capture the magnetic nano or micro particles as they flow by in the carrier medium. For example, the separation can take place within a laminar flow of a carrier fluid along a thin micro channel. A field gradient is imposed across the thin dimension of the channel, perpendicular to the direction of the flow.

An important difference between the use of a permanent magnet and an electromagnet is the much lower generated magnetic induction of the latter. A permanent magnet easily generates a magnetic induction of 0.5 - 1 T, while the magnetic induction of a simple planer coil is in the mTesla range.

Integrated electromagnet will produce a much smaller magnetic force, so that fluid flow in the micro channels needs to be strongly limited or that magnetic beads need to pass at a very close distance with the planar coil.

Retention and elution of the magnetic particles is a strong function of their magnetic moments, as stronger magnetic particles form thin clusters near the capillary wall with less interaction with the parabolic flow profile induced by a pressure gradient, while magnetically weaker particles feel the full viscous drag from the centre of the flow, resulting in an effective separation.

**Magnetic Bead Transport**

In magnetic transport, magnetic forces effectively transport the particles: it requires magnetic fields and magnetic forces that act on a larger range than necessary for separation, where magnetic beads approach very closely the magnetic actuation region by the fluid motion. Manipulation of magnetic beads in general and transport in particular is a difficult task, as the magnetic susceptibility X of the magnetic beads is rather weak (typically x $\leq$ 1), due to the small magnetic volumes of the particles. This explains why mostly the large field of (mechanically moving) permanent magnets has been used for the separation, transport and positioning of magnetic microbeads. In an approach towards miniaturization and automation of analytical applications, a system has been proposed in which liquid movement is substituted with magnetically induced movement of magnetic particles. Fluidic channels were realised on a plastic cartridge of centimetre size and magnetic transport was induced by mechanically moving external permanent magnets. In another approach, magnetic particles have been transported over millimeter distances in a microfluidic channel using an array of electromagnets actuated in a four-phase scheme. Each electromagnet consisted of a 0.3 mm diameter magnetic needle core with a wire-wound coil of 300 turns. For coil currents of the order of 0.5 A, forces of 0.1 pN were demonstrated.

Also miniaturized magnetic solutions have been proposed for bead transport, thereby taking full profit from batch microfabrication technologies. Typically, the size of the micropatterned magnets determines the spatial range, where appreciable magnetic forces acting on the microbeads exist.

Serpentine gold wires micropatterned on silicon substrates have been combined with microfluidic structures realized in PDMS to transport 4.5 $\mu$m polystyrene-coated magnetic beads. By engineering the magnetic field produced by different current-carrying wires, a microsystem was realized that could generate local magnetic field maxima that trap the magnetic beads. When the field maxima change locations, the microbeads follow those maxima. The device allowed precise positioning and transport over 100 mm distances in a single actuation event, which is partly due to the presence of a permanent magnet placed in proximity of the microfluidic chip, the role of which principally is to enhance the magnetic force by inducing a magnetic moment in the magnetic beads.

A micro-electromagnet wire matrix, based on two layers of mutually orthogonal arrays of linear wires, has demonstrated magnetic transport of 1-2 $\mu$m size magnetic particles over 20 mm distances in a single actuation event.

This is a typical working range for the magnetic force, generated by a current-carrying conductor, when no external permanent magnet is used to induce a magnetic moment to the beads. Transport using current carrying wires or microcoils in combination with a magnetic substrate or film to enhance the magnetic forces has been demonstrated more recently.

In another approach, a simple planar coil array-based magnetic transport system has been proposed, in which an individual coil is capable of displacing beads over millimeter distances in a liquid-containing capillary. A drastic increase of the magnetic energy and magnetic forces acting on the beads was obtained by placing the complete coil array in a uniform static magnetic field that imposes a permanent magnetic moment to the microbeads. The very small magnetic field gradient of a simple planar coil proved then to be sufficient to displace 1 $\mu$m diameter beads over a distance of the order of the coil size. The coils were realized using simple printed circuit board (PCB) technology (100 $\mu$m Cu winding width, 35 $\mu$m winding height, 200 $\mu$m winding pitch) and had a small number of windings (N = 4$^{-10}$).

A single coil typically generates a magnetic field gradient of about 5 mT (mm)$^{-1}$ for a maximum allowed current density of 400 A (mm)$^{-2}$. Arranging adjacent coils with spatial overlap over two layers of the PCB circuit and actuating them in a specific three-phase scheme assured the long-range displacement of the microbeads. Moreover, it was found that these polarized beads formed columns with a length of the order of the microfluidic channel size, due to magnetic dipole interactions. This column formation was partly at the basis of the strong magnetic force due to the larger magnetic volume and lower demagnetization factor of the structure.

**Magnetic Beads as labels for detection**

Detection of biological molecules is usually accomplished using biomolecular recognition between the target molecule and a specific receptor (e.g. an antibody) that is tagged with a label. The label may be a radioisotope, enzyme, fluorescent molecule or charged molecule, for example. Recently, also magnetic beads have been used as labels for biosensing.

Magnetic labels have several advantages over other labels. The magnetic properties of the beads are stable over time, in particular, because the magnetism is not affected by reagent chemistry or subject to photo-bleaching (a problem with fluorescent labels). There is also no significant magnetic background present in a biological sample and magnetic fields are not screened by aqueous reagents or biomaterials. In addition, magnetism may be used to remotely manipulate the magnetic particles. A number of very sensitive magnetic field detection devices have been developed during recent years, like giant magnetoresistance (GMR) and spin-valve magnetic sensors that enable the measurement of extremely weak magnetic fields, for example the magnetic field generated by the magnetization of a magnetic particle. A basic GMR unit element or spin-valve device consists of a pair of magnetic films separated by a non-magnetic conducting layer.

When an external magnetic field rotates the magnetizations of the magnetic layers towards alignment, spin-dependent electron scattering is reduced at the interfaces within the device, decreasing its electrical resistance. GMR sensors can be microscopic in size and sensitive to the presence of micron and smaller size magnetic particles, when in close proximity.

Besides GMR sensors, measurements of single magnetic beads have been demonstrated using miniaturized silicon Hall sensors and planar Hall effect sensors based on permalloy thin films.

Researchers at the Naval Research Laboratory, Washington DC, followed by others, have developed microsystems for the capture and detection of micronsized, paramagnetic beads on a chip containing GMR or spin-valve sensors. The technology is mostly based on a sandwich assay, where the target molecule is bound to an immobilized probe on the GMR sensor, after which the magnetic label is bound to the target using specific ligand-receptor interactions. A magnetic field gradient can be applied to attract magnetic beads to the chip or to selectively pull off only those beads not bound to the surface by specific binding.

The field can be generated by external permanent magnets or electromagnets, electromagnets integrated with the chip or by current carrying lines positioned close to the chip. Bound beads are detected by the GMR sensors by applying a uniform magnetic field perpendicular to the substrate, imposing a magnetic moment to the superparamagnetic beads. This induced moment generates an in-plane magnetic field that is measured by the GMR sensor. Applying the uniform field normal to the plane of the GMR sensor rather than in-plane has the advantage that, due to demagnetization effects, a much larger magnetizing field can be applied to the beads without saturating the sensor.

**Separation and mixing using magnetic supraparticle structures**

When placed in a magnetic field, super paramagnetic particles will acquire a magnetic moment and they will start interacting by the magnetic dipole interaction. This interaction induces a spontaneous clustering of the particles into larger, often complicated, structures, sometimes called magnetic supraparticle structures (SPS). The shape of an SPS depends on parameters, like the size of the magnetic moment of the microbeads and the magnetic dipolar interaction between different beads. These properties are dependent on the amplitude and frequency of the applied magnetic field, the shape and magnetic content of the beads, the concentration of the magnetic particles in the fluid, the temperature etc. (Hayes, M.A. et. al. (2001) Active control of dynamic supraparticle structures in microchannels, Langmuir 17 (9) 2866 -2871, and Rida, A. et. al (2004) Dynamics of magnetically retained supraparticles structures in liquid flow, Applied Physics Letters 85:4986-4988)Magnetic particles based assay has became a standard in modern chemical and biological diagnostics. However, the current particles handling systems present very limited capabilities. This is an important issue as the diagnostics test procedures are becoming more and more complex. On the other hand, "micro"-fluidic based technology is nowadays perceived as an emerging technology with great potential that can lead to more simple integration of complex testing procedures in an easy-to-use device. However, the manipulation of magnetic particles in a fluidic environment is facing a physical constraint: to be compatible with a "continuous" fluidic processing environment, the particles must be continuously exposed to a magnetic field. Under such conditions the particles will stick together and agglomerate, thereby losing their main advantage: the particle active / coated surface that is in contact with surrounding bio-chemical liquid will be drastically reduced which will seriously compromise the assay performance. Different attempts to solve those issues have been performed (WO2008/116543; WO03/061835; WO2004/078316; WO2008/010111; EP19970116857 and WO/03086637). Most of those technologies are not suited for a small point of care (POC) instrument simply because of either lack of simplicity which makes them expensive to produce. Another disadvances with the above referred methods is the use of electromagnet which is not suited for small POC instrumentation due to high power consuming units with relative low magnetic power as discuss elsewhere in this application.

## Magnetic Beads as surface area for immuno and DNA assays

Immuno-assays are probably the most important analysis method for biological molecules, as the molecular recognition reaction provides high selectivity and chemical sensitivity. Although conventional microtiter plate assays continue to play an important role, enzyme assays, DNA binding and competitive immuno-assays have been performed on microdevices.

Heterogenous assays, where reactions occur both in solution and in a solid phase, offer the advantage of easy separation of chemical complexes from reactants. Bio-molecule immobilization on a solid phase, formed by the surface of micro- or nanoparticles, evidently results in a small-volume and localized assay. Such solid phase provides a high surface to-volume ratio, reducing diffusion times during the microfluidic procedures and increasing the density of binding sites, which is beneficial for a high detection signal and sensitivity. In addition, such assay allows for a rapid regeneration and exchange of the solid support when needed.

The microfluidic procedure involved antigen capture from the sample solution to the beads, rinsing, elution and electrophoretic separation of the sample. The beads could be replaced after each run, eliminating the need to regenerate the solid support.

In another study, a small-volume heterogeneous immuno-assay was demonstrated using both classical fused silica capillaries and glass microfluidic chips. The system demonstrated the presence of parathyroid hormone at concentrations of 1 $\mu$g/L, using a sandwich assay, which is based on the trapping or capture of the analyte by one antibody and the detection by another (fluorescent) antibody. Also a calibration curve was generated at clinically relevant levels (2-50 $\mu$g/L) for the detection of interleukin-5, an important cytokine for asthma, immune response cascades and cancer. Dynamic DNA hybridization was demonstrated by immobilizing DNA targets on magnetic beads via streptavidin-biotin conjugation or base pairing between oligonucleotide residues. The DNA/bead complex was introduced into the device, after which hybridization took place with a complimentary probe. The hybridized probe was then removed by heat denaturation to allow the DNA sample to be interrogated again by another probe with a different sequence of interest. Demonstration of specific hybridization reactions in an array format was achieved using four synthesized DNA targets and five probes in sequence, indicating the potential of this approach for gene expression analysis.

The ability to manipulate magnetic beads on a chip surface using different test structures further revealed the potential of the dynamic magnetic biosensor approach for a broad spectrum of lab-on-a chip applications, where single or a small amount of bio-molecules attached to magnetic particles are to be guided to specific places on the chip.

Based on the description above it can be concluded that a major concern when quantitatively detecting the presence or absence of analytes in especially small samples is the elimination or reduction of background signal arising from clustering of paramagnetic particles, which heavily disturbs the reliability and reproducibility of detecting small amounts of analyte. Further the clustering decreases both the accuracy and the precision of a given assay as well as shielding the signal from the single paramagnetic particles, which further reduces the signal-to-noise ratio.

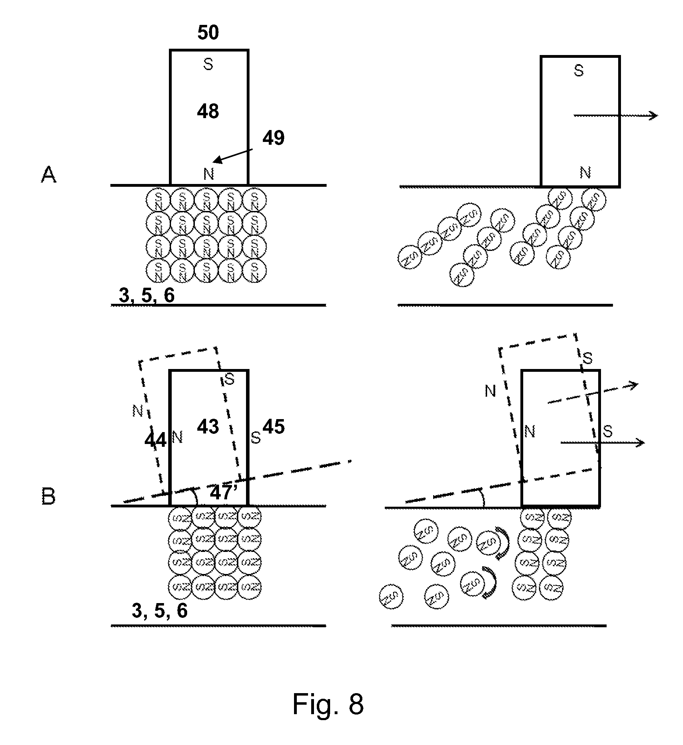

In the prior art the positioning of the means generating the magnetic field relative to the flow direction of the fluid comprising the paramagnetic material has been such that the poles of the magnetic field are both placed directly perpendicular to the flow direction of the liquid in the channel device. In other words the magnetic devices known in the art generate a magnetic field having first and second opposite poles arranged so that they are not mutually spaced viewed in the longitudinal direction of the capillary channel. An example of a prior art device is shown in fig. 8A. The positioning of the magnetic field in relation to the flow direction of the paramagnetic particles has not even been considered to be of any importance.

Disclosure of the Invention

The object of the present invention was to develop a system and a method for quantitatively detecting the presence or absence of a target analyte in a small liquid sample, wherein 1. accuracy and precision of a given assay is improved, especially for small quantities of analyte, 2. background unspecific signal is reduced or eliminated, and 3. the signal arising from the analyte is increased.

By combining microfluid and magnetic particle technology in a special constellation the present inventors found that it was possible to fulfil the critical parameters and at the same time apply the constellation in a relative small handheld instrument (below 500 gram), capable of analysing samples of less than 200μl.

Surprisingly, it was found that positioning the magnetic field relative to the flow direction of the fluid comprising the paramagnetic material was of great importance. Accordingly it was found that arranging the magnetic field such that the poles of the magnetic field are mutually spaced viewed in the longitudinal direction of the capillary channel, provided a significant improvement.

Accordingly the invention relates in one embodiment to a system for clustering and/or homogenously distributing a plurality of paramagnetic particles in a capillary channel. The system comprising a channel device and a magnetic device.

The channel device comprising a longitudinal capillary channel containing a plurality of paramagnetic particles.

The magnetic device comprising receiving means for receiving the channel device and comprising magnetic field generating means for generating a magnetic field. The magnetic field having first and second opposite poles and are arranged so that its poles are mutually spaced viewed in the longitudinal direction of the capillary channel. Ideally the mutual spacing is such that the magnetic field lines are substantial perpendicular to the flow direction in the channel. The magnetic generating means are arranged so that the paramagnetic particles are subjected to the field. Further the magnetic device comprises means for changing the magnetic field.

In another embodiment the field changing means comprises means for applying and removing the magnetic field.

In another embodiment the field changing means comprises means for moving the field generating means longitudinally in relation to the capillary channel.

Further the invention relates to a method for clustering or homogeneously distributing a plurality of paramagnetic particles contained in a liquid in a longitudinal capillary channel. The method comprising:

> a) solution of a layer comprising the plurality of paramagnetic particles with the liquid through the first end (21) of the channel;
>
> b) applying a magnetic field to at least a portion of the channel by way of a magnetic field generating means (43) having first and second opposite poles. The magnetic field generating means being arranged relatively to the channel so that the poles thereof are mutually spaced viewed in the longitudinal direction of the channel, thereby attracting at least some of the particles to the inner surface of the channel, thereby forming clusters of the particles;
>
> c) moving the magnetic field generating means longitudinally in relation to the channel, and at a chosen distance and at a suitable velocity, where the total resulting forces on the particles results in the clusters of particles displace in direction opposite the direction of the magnetic field generating means, thereby the particles will shift magnetic polarity and released as smaller clusters and single particles and by gravity distributed homogeneously on the bottom of the channel over the chosen distance;
>
> d) optional repeating step c where the magnetic field is moved in the reverse longitudinal direction; and
>
> e) removing the magnetic field.

In another embodiment the invention relates to a method for quantitative detection of the presence or absence of a target analyte in a sample consisting of less than 200 μl liquid, comprising the steps of:

> a) providing an analyte containing liquid sample consisting of less than 200μl liquid;
> b) supplying the liquid analyte containing sample to a first end (21) of a capillary channel and thereby solution of a layer as defined in step a) of the method of the embodiment above, the capillary channel comprising a first reaction part (3) and a second detection part (5, 6), the two parts being physically separated such that sample material cannot enter into contact with the second detection part prior to step j) below;
> c) contacting the sample with an immobilisation matrix comprising the paramagnetic particles and capable of capturing the analyte;
> d) binding of the analyte to the immobilisation matrix in the first reaction chamber (3) by way of step c)-d) according to the method of the embodiment above;

e) optional repeating step d) one or more times;

f) immobilising the immobilisation matrix comprising the captured analyte as defined in step b) of the method of the embodiment above;

g) discarding the supernatant;

h) supplying a washing solution and washing the chamber as defined in step c)-d) of the method of the embodiment above, discarding the washing solution;

i) optional repeating step h) one or more times;

j) transferring the immobilisation matrix comprising the captured analyte to the second detection part (6) of the chamber as defined in step c) according to the method of the embodiment above; and

k) releasing the immobilisation matrix by way of step e) of the method according to the embodiment above and detecting the presence or absence of a target analyte by using conventional detection means.

In another embodiment the invention relates to a method further comprising a step b') or c') of contacting the analyte with a signal generating biological marker capable of binding to the analyte.

In another embodiment the invention relates to a method, where the signal generating biological marker comprising an antibody.

In another embodiment the invention relates to a method, where the signal generating biological marker comprising HRP (Horseradish Peroxidase) or ALP (Alkaline Phosphatase) conjugated to the biological marker.

In another embodiment the invention relates to a method, where the step b') is performed after step b) and prior to step c).

In another embodiment the invention relates to a method, where the step c') is performed after step c) and prior to step d).

Brief Description of the Drawings

The invention is explained in detail below with reference to the drawings, in which

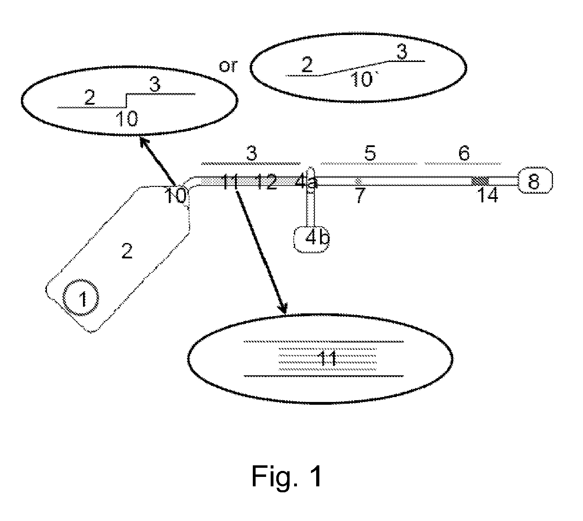

Fig. 1 illustrates a schematic presentation of a sample device comprising a microfluid channel having a first part (3) and a second part (5, 6), an application zone (1), a separation chamber (2), a first capillary channel (3), a collection chamber (4a), a waste outlet (4b), a washing chamber (5), a detection chamber (6), paramagnetic particles (7) (which may be transferred between the first and the second part) located in washing chamber, an inlet channel for washing and detector solution (8), a physical barrier (10 (vertical), 10' (incline)) between the separation chamber and the first capillary channel, hydrophilic coating (11) in the first capillary channel (3), corona treatment (12) (symbolised by the grey shade) of the first capillary channel, and a detection window (14). When starting the assay the magnetic particles are situated in the first part (3).

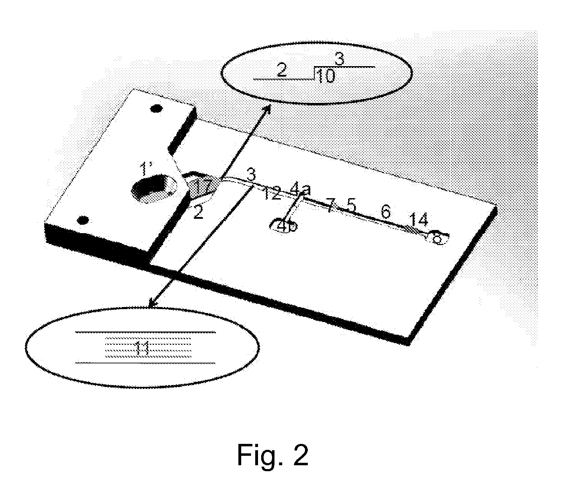

Fig. 2 illustrates the same principle as in Fig. 1 with a three dimension illustration.

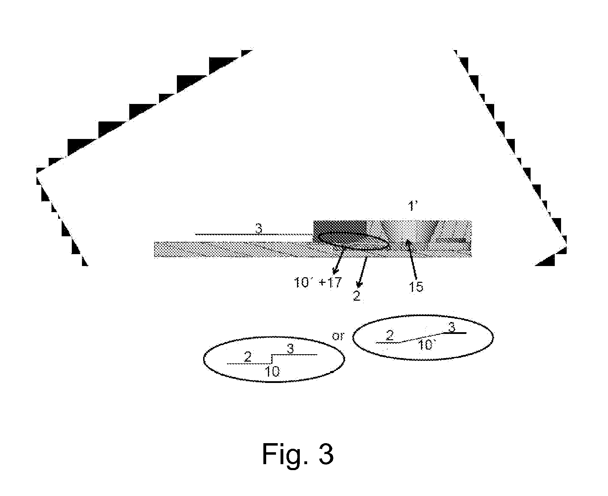

Fig. 3 illustrates a schematic site view of a separation device comprising a microfluid channel (3), an application well (1'), a separation chamber (2), a first capillary channel (3), a physical barrier (10') between the separation chamber and the first capillary channel, a hydrophilic filter material (17), and a prefilter (15).

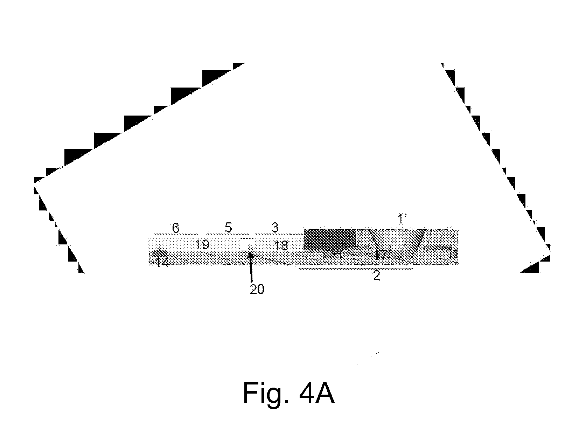

Fig. 4a illustrates a schematic site view of an integrated separation and detection device comprising a microfluid channel (3,5,6), an application well (1), a separation chamber (2) and a hydrophilic filter (17), a first capillary channel (3), serum/plasma (18) in the first capillary channel, signal solution (19) in washing (5) and detector chamber (6), light trap version A (20) in connecting junction between the first capillary channel (3) and the washing chamber (5), and a detection window (14).

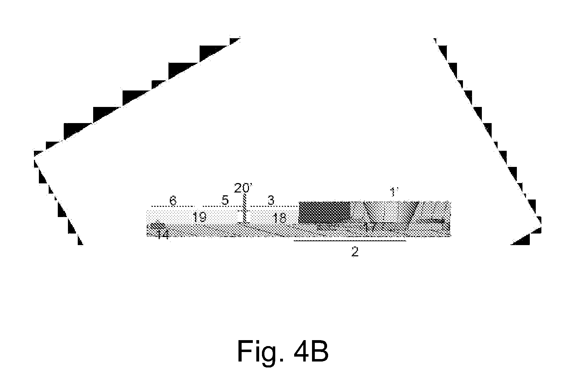

Fig. 4b illustrates a schematic site view of an integrated separation and detection device comprising a microfluid channel (3,5,6), a application well (1), a separation chamber (2) and a hydrophilic filter (17), a first capillary channel (3), serum/plasma (18) in the first capillary channel, signal solution (19) in washing (5) and detection chamber (6), a light trap version B (20') (e.g. by introducing a bend on the path from the first part to the second part of the chamber, so the exit point from the first part and the entry point of the second part in different levels) in connecting junction between the first capillary channel (3) and the washing chamber (5), and a detection window (14).

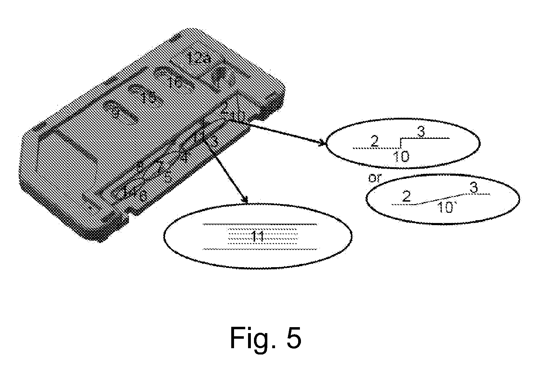

Fig. 5 illustrates same principle as in fig. 1 with a three dimension illustration including more features. An integrated separation and detection device comprising a microfluid channel having three compartements (3, 5, 6), an application well (1'), a separation chamber (2), a first capillary channel (3), a collection chamber (4) with a waste outlet, a washing chamber (5), a detection chamber (6), magnetic particles location in washing chamber (7), an inlet channel for washing and detector solution (8), a physical barrier (10, 10') between the separation chamber and the first capillary channel, hydrophilic coating (11) in the first capillary channel (3), a detection window (14), a first compartment for detection solution A (9), a second compartment for detection solution B (15), a washing solution compartment (16), and a blood lid (12a).

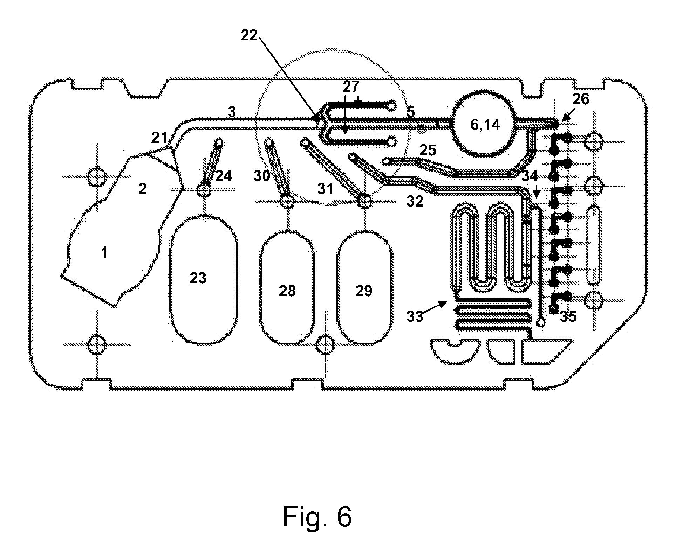

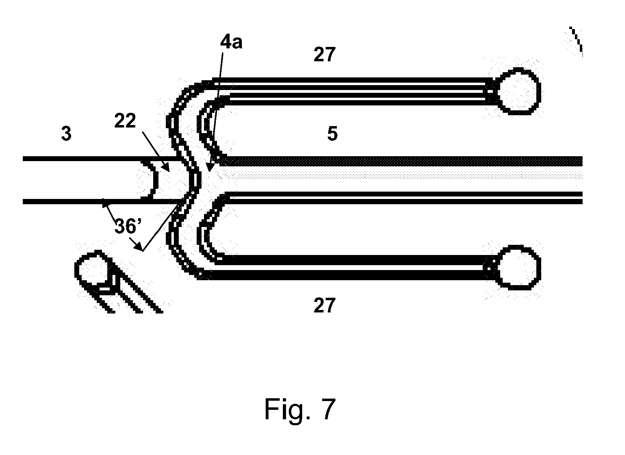

Fig. 6 illustrates a top view of an integrated separation and detection device comprising an application well (1), a filtration area (2), a plasma inlet (21), a first part channel (3) connected to the absorbing barrier and capillary stop (22). A blister container with washing solution (23) is connected to the microfluid system via channel (24) connected to channel (25) and into the detection area via channel (26) and (6). The washing channel (5) ends in the collection chamber (4a at fig. 7) (at the capillary stop (22)), where it is connected to two side channels (27), which end in a waste container (not shown). In the washing channel, there is a detection area (window) (6, 14). Blister (28) is connected to channel (30) and blister (29) is connected to channel (31). The channels (30) and (31) are connected to channel (32), which are connected to channel (33), when signal solutions from reaches channel (33), the remaining signal solutions enter channel (34) and are mixed in channel (35), which is connected to the plasma channel at point (26).

Fig. 7 illustrates a schematic top view of the area of the capillary stop (22), the collection chamber 4a, the two side channels (27) as described in fig. 6., and the first angle (36').

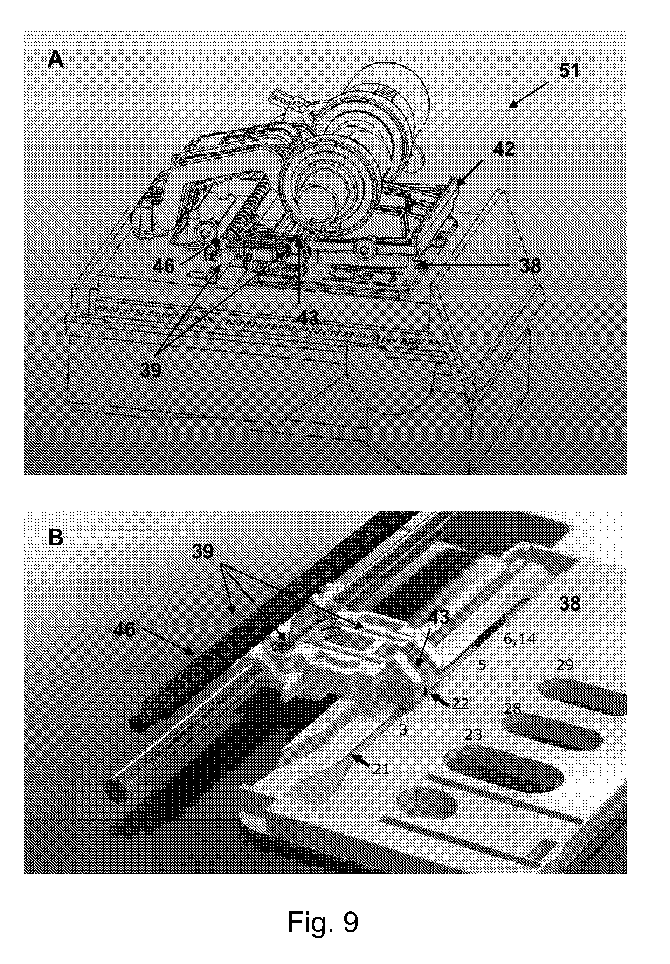

Fig. 8 illustrates the principle of the invention (B) with the magnetic field lines substantially perpendicular to the flow direction in the capillary channel (3, 5, 6) compared to a situation where the magnetic field lines are substantially parallel to the flow direction (A).
The substantially parallel field lines in (A) is obtained by a magnetic field generating means (48) having first (49) and second (50) opposite poles, where the magnetic field generating means being arranged relatively to the channel so that the poles thereof are not substantial spaced viewed in the longitudinal direction of the channel.
The substantially perpendicular field lines in (B) is obtained by a magnetic field generating means (43) having first (44) and second (45) opposite poles, where the magnetic field generating means being arranged relatively to the channel so that the poles thereof are mutually spaced viewed in the longitudinal direction of the channel. The mutual spacing of the poles seen in the longitudinal direction of the channel is defined by a first angle (47') to the channel. Preferably the angle is between 0 and 20 degrees, and most preferably about 0 degrees.
The field changing means comprises means (46, see fig. 9) for moving the field generating means longitudinally in relation to the capillary channel of the channel device. When the magnetic field is applied to at least a portion of said channel by way of the magnetic field generating means (43) paramagnetic particles are attracted to the inner surface of the channel, thereby forming clusters of particles. When moving the magnetic field generating means longitudinally (indicated by the arrows) in relation to the channel, at a chosen distance and at a suitable velocity, where the total resulting forces on the particles results in the clusters of particles displace in direction opposite the direction of the magnetic field generating means. In (A) the particles will not shift magnetic polarity (indicated by the half circular arrows) and be released as rows/clusters of particles. In (B) the particles will shift magnetic polarity and released as smaller clusters and single particles and by gravity distributed homogeneously on the bottom of the channel over the chosen distance.

Fig. 9 (A) is a side-top-front view of a analysis apparatus (51) including the system comprising a magnetic device (39) comprising movable receiving means (42) for receiving the channel device (38) and the magnetic field generating means (43).
(B) is a side-top-front view of the system comprising a magnetic device (39) comprising receiving means (42) for receiving the channel device (38) and the magnetic field generating means (43).

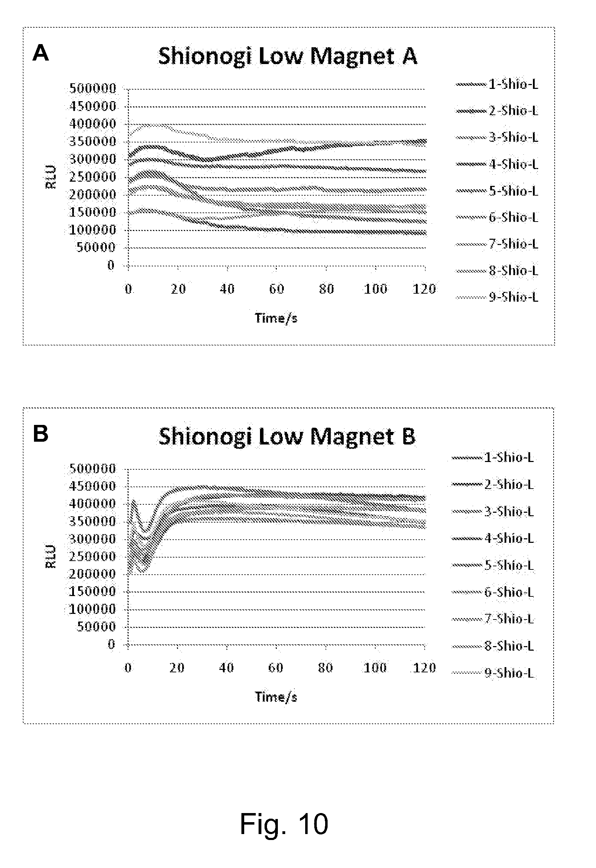

Fig. 10. (A) and (B) illustrates the RLU values from the experiments A and B in the example, respectively.

**Definitions**

In the context of the present invention, by "paramagnetic" and "superparamagnetic" is meant magnetism which occurs only in the presence of an externally applied magnetic field. Unlike ferromagnets, paramagnets do not retain any substantial magnetization in the absence of an externally applied magnetic field, because thermal motion causes the spins to become randomly oriented without it. Thus the total magnetization will in theory drop to zero when the applied field is removed. Further superparamagnetic materials follows the Curie type law but with exceptionally large values for the Curie constants.
In the context of the present invention, by "homogeneously distributing" is meant a distribution where most of the particles in a cluster of the particles primarily (preferable) is released as single particles and secondarily in smaller clusters separated substantially homogenously over a given area of a capillary channel.
In the context of the present invention, by "total resulting forces" is meant the sum of forces affecting the paramagnetic particles when applying the magnetic field, during moving of the magnetic field and after removal of the magnetic field. The total resulting forces comprise:

1) Horizontal forces: hydrodynamic drag force when moving the magnetic field along the capillary channel. The hydrodynamic drag force depends on the viscosity of the liquid in the capillary channel and of the velocity of the movement of the magnetic field.

2) Vertical forces: magnetic field strength and gravity.

3) Other forces: Van der Waals forces aligned perpendicularly to the inner wall of the capillary channel.

In the context of the present invention, by "capillary channel" is meant a narrow tube or channel through which a fluid can pass. Preferably the diameter (or with) of a capillary channel according to the invention is less than 10 mm. Even more preferred the diameter of a capillary channel according to the invention is less than 5mm, such as less than 4 mm, or less than 3 mm or even less than 2 mm. In a most preferred aspect the capillary channel has a diameter of 1 mm or less, e.g. 0,2-1.0 mm. The channels may also be formed of non-circular shapes, e.g. rectangular or triangular, in which case the "diameter" refers to the mean distance from the center of the cannel to the periphery.

The terms "capillary channel" and "first capillary channel" are used interchangeable.

In the context of the present invention, by "micro channels" or "capillary micro channel" is meant a very small narrow tube or channel through which a fluid can pass. Preferably the diameter or with of a micro channel according to the invention is less than 1/5 of the capillary channel. Even more preferred the diameter of a micro channel according to the invention is less than 1 mm, such as less than 0.5 mm, or less than 0.2 mm or even less than 0.1 mm. In a most preferred aspect the mircro channel has a diameter of 0.1 mm or less, e.g. 0.02-0.1 mm. The channels may also be formed of non-circular shapes, e.g. rectangular or triangular, in which case the "diameter" refers to the mean distance from the center of the cannel to the periphery.

In the context of the present invention, by "lower part" is meant the part of a device when in use, which is closest to the center of the earth. By "upper" is meant the opposite, namely, the part furthest away from the centre of the earth when in use. Accordingly, a liquid would lie on the lower part and not the upper part when in use.

Detailed description of the Invention

In a first embodiment the invention relates to a system (37) for clustering or homogenously distributing a plurality of paramagnetic particles in a capillary channel. The system comprising a channel device (38) and a magnetic device (39);

a. The channel device comprising a top face (40) and a bottom face (41), a longitudinal capillary channel (3, 5, 6) being provided between the top face and the bottom face. The channel having an inner surface and a first end (21) connected to a sample inlet (1) and a second end, and the channel containing a plurality of paramagnetic particles; and

b. the magnetic device comprising receiving means (42) for receiving said channel device and comprising magnetic field generating means (43) for generating a magnetic field having first (44) and second (45) opposite poles. The magnetic generating means being arranged so that its poles are mutually spaced viewed in the longitudinal direction of the channel of the channel device received by the receiving means and so that the paramagnetic particles are subjected to the field; and the magnetic device comprises means for changing the magnetic field.

In another embodiment the field changing means comprises means for applying and removing the magnetic field.

In another embodiment the field changing means comprises means (46) for moving the field generating means longitudinally in relation to the longitudinal channel of a channel device received by the receiving means.

In a preferred embodiment the mutual spacing of the poles seen in the longitudinal direction of the channel is defined by a first angle (47') to the channel being lower than 89 degrees, preferably between 0 and 60 degrees, more preferably between 0 and 40 degrees, even more preferably between 0 and 20 degrees, or most preferably about 0 degrees

In another embodiment the magnetic generating means is either a permanent magnet or an electromagnet.

In a preferred embodiment the capillary channel has a cross section area of less than 1 mm$^2$, preferably 0.1-0.7 mm$^2$.

In one embodiment the width and height of the capillary channel is about 1.0 mm and about 0.2 mm, respectively. The length of the capillary channel from the outlet of the separation chamber (3) to the inlet of collection chamber (4a) is 5-50 mm. In one embodiment the length of the capillary channel from the outlet of the separation chamber to the inlet of collection chamber is 5-20 mm. In one embodiment the length of the capillary channel from the outlet of the separation chamber to the inlet of collection chamber is 12-20 mm.

In another embodiment the system is for the quantitative detection of the presence or absence of a target analyte in a liquid sample having a volume of less than 200 $\mu$l, wherein the paramagnetic particles in the channel of the channel device are comprised in an immobilisation matrix capable of capturing the analyte.

In a preferred embodiment the channel device of the system comprises:

a) a first part comprising the first part of the capillary channel (3) and the sample inlet (1) at the first end (21) for the introduction of a sample containing an analyte;

b) a second part (5, 6) comprising a washing zone (5) and a detection zone (6), said detection zone comprising a detection window (14) through which the target analyte can be detected by detection means;

c) a solution inlet (8, 25, 26) at the second end for introduction of washing solutions and reaction mixtures; and

d) a discharge outlet (4b, 27) for the discharge of waste products.

In a preferred aspect the internal width and height of the reaction chamber, or at least the first part of the reaction chamber, is 0.1-5 mm and 0,05 - 2 mm respectively . More preferably, the internal width and height of the reaction chamber, or at least the first part of the reaction chamber, is 0.25-2 mm and 0.2 - 1 mm, respectively

In a preferred embodiment the system comprises two or more channel devices as defined above.

In a preferred aspect the length of the reaction chamber is 2-30 mm, more preferably 5-20 mm.

In a further embodiment first and second parts are separated such that liquid sample material from the first part (3) of the chamber cannot enter the second part (5, 6) of the chamber.

In a further embodiment the paramagnetic particles are superparamagnetic particles.

In a preferred embodiment the first and second parts are separated by a collection chamber (4a). The collection chamber serves as a connection point for the waste outlet(s) (4b, 27) for waste products such as washing solution and residual sample material.

In one aspect the especially at least the lower part of the internal surface of the first capillary channel facing the liquid is a plastic material surface treated by oxidation, preferable by corona treatment. Thus it was observed by visual inspection that the capillary channel was very efficient in pulling the liquid into the capillary channel.

In a preferred embodiment the plastic material is polystyrene, polymethylmethacrylate, polyethylene, polypropylene, polyacrylates, silicon elastomers, acryl nitrile butadiene styrene, or the like.

However, to improve the stability of the hydrophilic surface it was found that it was necessary to coat the surface with a coating comprising a hydrophilic substance. Preferably, the hydrophilic substance is selected from the group comprising monosaccharides, disaccharides, aminosaccharides, surfactants, serum albumine and immunoglobulins.

This further coating process can be performed without or without the immobilisation matrix comprising the paramagnetic particles for the purpose of obtain a hydrophilic further coating.

The channel device according to the invention may be obtained by increasing the hydrofilicity of the plastic material of the capillary channel (e.g. by washing and corona treatment). Hereafter, a solution comprising the hydrophilic substance (e.g. monosaccharides, disaccharides, aminosaccharides, surfactants, serum albumine and immunoglobulins), preferably further comprising the immobilisation matrix, is dispensed into the capillary channel.

The purpose of wash is to get the plastic surface as free as possible from impurities in order to exclude impurities witch may interfere with the analytical result and maintain the hydrophilic effect of the corona treatment until coated with coating solution. The hydrophilic effect of corona treatment was observed to diminish with time. The diminish effect is to a large extent dependent on the purity of the plastic. The hydrophilic effect of the corona treatment for non transparent capillary channel was observed to be short (typically hours to days).

Preferably, the coating solution comprises the immobilisation matrix, whereby the further benefits of keeping the immobilisation matrix easily soluble and protected from aggregation is achieved. Further, this protect labels on the immobilisation matrix (storage stability) and keep the channel hydrophilic for long time (months-years) so sample channel easily and fast can be filled.

Another benefit observed was an increased drag force of the treated capillary channel. Further, a good and even distribution of immobilisation matrix ensures to initially get good contact between sample and immobilisation matrix and further ensure an almost complete recovery of the paramagnetic particles when collected with a magnet.

In a preferred aspect, the capillary channel comprises a stop zone (22) where the liquid sample material is not dragged further. This stop zone may be formed by widening the capillary channel, and may e.g. be formed by a junction to a collection chamber. Preferably the stop zone is not coated with the hydrophilic substance.

In a further embodiment the coating further comprises an immobilisation matrix. The immobilisation matrix is thereby stabilised and further the mixing of the sample material and the immobilisation matrix is improved.

Preferably, the immobilisation matrix comprises paramagnetic or superparamagnetic material.

In a preferred aspect of the invention the first and second parts are separated such that a significant part of the signal (e.g.light) may not be transferred from the first part of the chamber to the detector part of the second part of the chamber. By a significant part is meant more than 50%, such as more than 75% or even more than 90%, or even more than 99%. This may be achieved by placing the exit point from the first part and the entry point of the second part in different levels e.g. by introducing a bend on the path from the first part to the second part of the chamber, such that signal (in the form of light rays) from the first part of the chamber may not enter the detection part of the second chamber. Another possibility is introducing a bend in the second part of the chamber such that the detector part is not in line with the entry point of the analyte to the second part of the chamber. A preferred possibility is the placement of a light-impermeable barrier between the two parts such that a significant part of the light is prevented from entering the second part from the first part. Of course the barrier must not prevent the transfer of analyte (e.g. via magnetic particles) from the first and second parts.

In a further preferred embodiment the first and second parts are separated such that light may not be transferred from the first part of the chamber to the detector part of the second part of the chamber.

In another embodiment the surface structure and the colour of the internal surface of the reaction chamber is light non-reflecting and/or light absorbing, respectively. The light non-reflecting and/or light absorbing surface may be obtained by obscuring and/or darkening of the surface. Optional the darkening is blackening, and preferable the colour of the internal surface of the reaction chamber is black.

In another embodiment the means for detection of the target analyte are selected among surface acoustic wave (SAW) detectors, spectrophotometers, fluorometers, CCD sensor chip(s), CCOS sensor chip(s), PMT detector(s), or any suitable light detector.

In a further aspect the invention concerns use of the system for the quantitative detection of the presence or absence of a target analyte in a sample.

In one embodiment the sample is derived from blood, preferable the sample is serum or plasma. Plasma may be obtained by applying an anti coagulant to the blood sample to be analysed. Preferred anti-coagulant may be selected among the group comprising K3-EDTA, citrate and heparine.

In preferred embodiments the magnetic field generating means is substituted by a rotating permanent magnet, or an electromagnet where the poles are switched, during the application and moving of the magnetic field.

In a further aspect the invention relates to a method for clustering or homogeneously distributing a plurality of paramagnetic particles contained in a liquid in a longitudinal capillary channel (3, 5, 6) of a channel device (38), said channel having an inner surface and a first end (21) connected to a sample inlet (1) and a second end with a solution inlet (8, 25, 26), said method comprising:

    a) solution of a layer comprising the plurality of paramagnetic particles with the liquid through the first end of the channel;

    b) applying a magnetic field to at least a portion of said channel by way of a magnetic field generating means (43) having first and second opposite poles, said magnetic field generating means being arranged relatively to the channel so that the poles thereof are mutually spaced viewed in the longitudinal direction of the channel, thereby attracting at least some of the particles to the inner surface of the channel, thereby forming clusters of the particles;

    c) moving the magnetic field generating means longitudinally in relation to the channel, and at a chosen distance and at a suitable velocity, where the total resulting forces on the particles results in the clusters of particles displace in direction opposite the direction of the magnetic field generating means, thereby the particles will shift magnetic polarity and released as smaller clusters and single particles and by gravity distributed homogeneously on the bottom of the channel over the chosen distance;

    d) optional repeating step c where the magnetic field is moved in the reverse longitudinal direction; and

    e) removing the magnetic field.

In a further aspect the magnetic generating means is either a permanent magnet or an electromagnet, preferable a permanent magnet.

In a preferred embodiment the mutual spacing of the poles seen in the longitudinal direction of the channel is defined by a first angle (47') to the channel being lower than 89 degrees, preferably between 0 and 60 degrees, more preferably between 0 and 40 degrees, even more preferably between 0 and 20 degrees, or most preferably about 0 degrees.

In a preferred embodiment the capillary channel has a cross section area of less than 1 mm$^2$, preferably 0.1-0.7 mm$^2$.

In one embodiment the width and height of the capillary channel is about 1.0 mm and about 0.2 mm, respectively. The length of the capillary channel from the outlet of the separation chamber (3) to the inlet of collection chamber (4a) is 5-50 mm. In one embodiment the length of the capillary channel from the outlet of the separation chamber to the inlet of collection chamber is 5-20 mm. In one embodiment the length of the capillary channel from the outlet of the separation chamber to the inlet of collection chamber is 12-20 mm.

In a preferred embodiment at least 90%, preferably 95%, more preferably 99% and most preferably 100% of the particles are attractable by the magnetic field.

In another embodiment the paramagnetic particles are superparamagnetic particles. Preferable the paramagnetic particles are comprised in an immobilisation matrix.

In a preferred embodiment the invention relates to a method for quantitative detection of the presence or absence of a target analyte in a sample consisting of less than 200 µl liquid, comprising the steps of:

a) providing an analyte containing liquid sample consisting of less than 200μl liquid;

b) supplying the liquid analyte containing sample to a first end (21) of a capillary channel and thereby solution of a layer as defined in step a) of the method of the embodiment above, the capillary channel comprising a first reaction part (3) and a second detection part (5, 6), the two parts being physically separated such that sample material cannot enter into contact with the second detection part prior to step j) below;

c) contacting the sample with an immobilisation matrix comprising the paramagnetic particles and capable of capturing the analyte;

d) binding of the analyte to the immobilisation matrix in the first reaction chamber (3) by way of step c)-d) according to the method of the embodiment above;

e) optional repeating step d) one or more times;

f) immobilising the immobilisation matrix comprising the captured analyte as defined in step b) of the method of the embodiment above;

g) discarding the supernatant;

h) supplying a washing solution and washing the chamber as defined in step c)-d) of the method of the embodiment above, discarding the washing solution;

i) optional repeating step h) one or more times;

j) transferring the immobilisation matrix comprising the captured analyte to the second detection part (6) of the chamber as defined in step c) according to the method of the embodiment above; and

k) releasing the immobilisation matrix by way of step e) of the method according to the embodiment above and detecting the presence or absence of a target analyte by using conventional detection means.

In a further embodiment the first (3) and second (5, 6) parts are separated by a collection chamber (4a). The collection chamber serves as a connection point for the waste outlet(s) (4b, 27) for waste products such as washing solution and residual sample material.

In a preferred embodiment the method further comprises a step b') or c') of contacting the analyte with a signal generating biological marker capable of binding to the analyte. Optionally the signal generating biological marker comprises an antibody.

In a preferred aspect of the invention the biological marker is one [or more] selected from compounds, mono-, oligo- and polyclonal antibodies, antigens, receptors, ligands, enzymes, proteins, peptides and nucleic acids. Preferably the biological marker is one or more selected from the group having the properties of light absorption, fluorescence emission, phosphorescence emission, or luminescence emission.

In a preferred embodiment the signal generating biological marker comprising HRP (Horseradish Peroxidase) or ALP (Alkaline Phosphatase) conjugated to the biological marker.

In a preferred embodiment the step b') is performed after step b) and prior to step c).

In a preferred embodiment the step c') is performed after step c) and prior to step d).

In a preferred embodiment the immobilisation matrix comprises an antibody capable of binding the analyte.

In a preferred embodiment the system comprises two or more channel devices as defined above.

In preferred embodiments the magnetic field generating means is substituted by a rotating permanent magnet, or an electromagnet where the poles are switched, during the application and moving of the magnetic field.

In a further aspect the invention relates to a separation device connected to one or more capillary channel(s) and comprising an upper part and a lower part, where the two parts when assembled form a separation chamber (2) comprising an application well (1') and a hydrophilic filter (17), one or more capillary channel(s) (3), said upper part having an inlet leading to the separation chamber.

It has further been shown during the course of the experiments leading to the present invention that by using or creating microchannels in the capillary channels, these were much more efficient in providing the required drag force on the liquid sample.

Accordingly, In one aspect the invention relates to a device for separating a suspension comprising 200 μl or less into a liquid phase and a retentate phase, said device comprising an application chamber (1) comprising a hydrophilic filter material (17), said application chamber being connected to one or more capillary channel(s) (3) each comprising two or more capillary microchannels.

In a further aspect the capillary channel comprises two or more capillary microchannels.

In a further aspect the capillary channel comprises three or more capillary microchannels.

Preferably, the micro channels are situated in the lower part of the capillary channel.

An object of the invention was to develop a device and a method capable to separate a suspension into a liquid phase and a retentate phase in a short time, where the liquid phase is substantially free of retentate contamination.

A further object was to develop a device and a method capable to separate a suspension into a liquid phase and a retentate phase in a short time where the separation is driven without the use of an external force.

This was achieved by the device according to the invention.

In a preferred aspect the sample to be analysed preferably has a volume of less than 200μl. In an even more preferred aspect the sample to be analysed has a volume of less than 150μl, even more preferred less than 100μl, even more preferred less than 90μl, such as less than 80μl, less than 70μl or even less than 60μl. In an even more preferred aspect the sample to be analysed has a volume of less than 50μl, even more preferred less than 45µl, even more preferred less than 40µl.

In a preferred aspect the first part of the capillary channel has a volume of less than 100µl. In an even more preferred aspect the the capillary channel has a volume of less than 90µl, even more preferred less than 80µl, even more preferred less than 70µl, such as less than 60µl, less than 50µl or even less than 40µl. In an even more preferred aspect the first part of the capillary channel has a volume of less than 30µl, even more preferred less than 25µl, even more preferred less than 20µl, such as less than 15µl, less than 10µl or even less than 5 µl.

**Examples**

Example, CV-values when detecting brain natriuretic peptide (BNP) in human plasma:

**Principle of the assay**

The BNP assay is a two-side sandwich immunoassay using direct chemiluminescence technology and constant amounts of two monoclonal antibodies. The first antibody (tracer) is a HRP-labelled monoclonal mouse anti-human BNP antibody specific to the ring structure of BNP (KY). The second antibody in the solid phase is a biotinylated monoclonal mouse anti-human antibody specific to the C-terminal portion of the BNP (BC203), which is coupled to streptavidin coated paramagnetic particles. A direct relationship exists between the amount of BNP present in the plasma sample and the amount of relative light units (RLUs) detected by the Photo Multiplier Tube (PMT, Hamamatsu).

**The channel device and pre treatment of the capillary channel**

18 channel devices (38) were fabricated in black and the detection window (14) of the device in clear ABS (acryl nitrile butadiene styrene) polymer. The overall length, height and thickness of the channel device were approximately 70, 35 and 2 mm respectively. The total length of the capillary channel (3, 5, 6) was approximately 40 mm, the width of the entire channel (3, 5, 6) was approximately 1.0 mm. The depth of the first part of the channel (3) was approximately 0.2 mm and the depth of all other parts (5, 6) of the channel was approximately 0.5 mm. The channel device was washed ultrasonically in a 50vol% water solution of 2-propanol and corona treated 25W/2s in order to increase the hydrophilicity prior to dispensing the paramagnetic particles.

After corona treatment, a solution of monoclonal mouse antibody BC203 (antibody against BNP) coated paramagnetic particles in a buffer (TBS: 0.05wt% Mouse IgG, 0.05wt% Bovine Gamma Gglobulin, 5 wt% sucrose, 5wt% BSA, 0.05vol% Tween, 1wt% Polyoxyetylene 10 tridecyl ether and 0.1wt% ProClin 300 (preservative)) was made and approximately 1µl of this solution was applied to the channel with a dispenser (Nanodrop ExtY) in such a way that only the first part (3) of the channel with exception of the capillary stop (22) area (about 1 mm in the longitudinal direction) was covered with the solution. The solution was left to dry up completely for approximately 15 minutes. The device was then sealed with a pressure sensitive adhesive film (PSA) and left for at least one day in order to recover the hydrophobicity of the capillary stop area and thereby creating a capillary stop.

**Conducting the assay**

Human plasma with a BNP concentration of 20 pg/mL (Shio-L) was added to a solution of monoclonal mouse BNP antibody "KY" covalently coupled to approximately one Horse Radish Peroxide molecule each. BNP and mouse antibody KY now specifically coupled in the solution. After approximately 1 minute of reaction time the human plasma solution was let in through the first end (21) and stopped by the hydrophobic functionality of the capillary stop (22). The plasma now dissolved the sugar matrix in the channel and thereby re-suspended the paramagnetic particles in the channel.

The 18 channel devices were divided in 2 groups of 9 each.

Either Magnet A (48) (group A) or Magnet B (43) (group B) was now put on top of the PSA and moved mechanically back and forth between the first end (21) and the capillary stop (22) in such a way that a minimum of paramagnetic particles was left in close contact with the magnet at the turning points.

In magnet A group the poles of the magnetic field are both placed directly perpendicular to the flow direction of the liquid in the channel device. In magnet B group the poles of the magnetic field are arranged so that they are mutually spaced viewed in the longitudinal direction of the capillary channel. The first angle (47') to the channel was 0 Degrees (fig. 8A and 8B).

This motion of the magnet was continued for approximately 200 seconds. During this period of time The BNP molecule formed a sandwich between the antibody KY and the antibody BC203 specific couple to the paramagnetic particles. The magnet was then moved slowly from the first end (21) to the capillary stop (22) in such a way that approximately 100% of the paramagnetic particles suspended were in close contact with the magnet as the capillary stop was reached.

A washing buffer (TBS: 0.05wt% BSA, 0.05vol% Tween, and 0.1wt% Kathon or 0.1wt% ProClin 300) was let in trough inlet (8, 25, 26) and filled the measuring area, the washing channel and approximately 50% of the waste channels. The magnet was then moved slowly from the capillary stop (22) to the washing channel (5) in such a way that approximately 100% of the paramagnetic particles suspended were in close contact with the magnet as the washing channel (5) was reached. The magnet was now moved back and forth between the ends (from (4a) to (6) and vice versa) of the washing channel (5) in such a way that a minimum of paramagnetic particles was left in close contact with the magnet at the turning points for approximately 20s. The magnet was now moved very fast away from the channel device and the paramagnetic particles fell via gravity to the bottom of the channel. The washing cycle was repeated once.

The magnet was then moved from the capillary stop to the center of the measuring area (14) of the detection part of the channel (6) in such a way that approximately 100% of the paramagnetic particles suspended were in close contact with the magnet as the center of the measuring area (14) was reached. A chemiluminescence substrate (Supersignal Elisa Femto Maximum Sensitivity Substrate from Pierce prod. 37074), which produces photons when brought in contact with HRP, was then let in through solution inlet (8, 25, 26) in order to completely fill up the measuring area (6, 14), the washing channel (5) and approximately 50% of the waste channels (27). The magnet was then moved back and forth between the ends (the entire leght of (6)) of the measuring area in such a way that a minimum of paramagnetic particles was left in close contact with the magnet at the turning points for approximately 8s. The magnet was now moved away very fast from the capillary channel and the paramagnetic particles precipitate via gravity to the bottom of the channel.

As soon as the magnet was moved away very fast from the measuring area, the PMT located under the measuring area was turned on and a measurement of the number of photons traveling through the window was performed for 120s.

The results of the two experiments are illustrated in Fig. 11 A and B and in table 1 below.

Table 1. Measured Relative Light Units (RLU) after 40 seconds of measurement expressed as the Mean Value, Standard deviation and CV value of the RLU's.

| | Magnet A | Magnet B |
|---|---|---|
| 1-Shio-Low | 279622 | 395732 |
| 2-Shio-Low | 308120 | 357968 |
| 3-Shio-Low | 215242 | 386774 |
| 4-Shio-Low | 110194 | 444728 |
| 5-Shio-Low | 171108 | 425300 |
| 6-Shio-Low | 171836 | 386988 |
| 7-Shio-Low | 179160 | 376776 |
| 8-Shio-Low | 134124 | 402616 |
| 9-Shio-Low | 355194 | 422506 |
| Mean Value | 213844 | 399932 |
| Std. | 78236 | 25435 |
| CV Value | 36,6% | 6,4% |

**Results and conclusion**

The results show that the signal was about 1.9 times higher in experiment B compared to experiment A and the standard derivation was about 3.1 times lower. This resulted in a remarkable smaller CV value (standard deviation/mean) about 5.7 times lower in experiment B compared to experiment A. The characteristics of the curve were also different for the two experiments. In experiment A the maximal RLU value was reached after about 7 s and remained at the maximal level until 15 s, resulting in a plateau at about 8 s. In experiment B the maximal RLU value was reached after about 20 s and remained at the maximal level until 40 s, resulting in a plateau at about 20 s.

All the mentioned characteristics reflect the expected differences in the behaviour of paramagnetic particles in clusters (experiment A) versus the behaviour of paramagnetic particles in smaller clusters and as single particles. Thus, in the latter situation the shield of light signals is expected to be smaller resulting in higher signal, the clustering of paramagnetic particles is lowered and the sedimentation of the paramagnetic particles is slower resulting in a longer plateau phase for the maximal RLU value.

This experiment clearly demonstrates the significant improvement of signal strength and CV value when using a magnetic field which is moved with its field lines substantially perpendicular to the flow direction compared to the situation where the field lines are substantially parallel to the flow direction.

**Claims**

1. System (37) for clustering and/or homogenously distributing a plurality of paramagnetic particles in a capillary channel, said system comprising a channel device (38) and a magnetic device (39);

   a. said channel device comprising a top face (40) and a bottom face (41), a longitudinal capillary channel (3, 5, 6) being provided between said top face and said bottom face, said channel having an inner surface and a first end (21) connected to a sample inlet (1) and a second end, and said channel containing a plurality of paramagnetic particles; and
   b. said magnetic device comprising receiving means (42) for receiving said channel device and comprising magnetic field generating means (43) for generating a magnetic field having first (44) and second (45) opposite poles, said magnetic generating means being arranged so that its poles are mutually spaced viewed in the longitudinal direction of the channel of the channel device received by the receiving means and so that the paramagnetic particles are subjected to the field; and said magnetic device comprises means for changing the magnetic field.

2. System according to claim 1, wherein the field changing means comprises means for applying and removing the magnetic field.

3. System according to claim 1 or 2, wherein the field changing means comprises means (46) for moving the field generating means longitudinally in relation to the longitudinal channel of a channel device received by the receiving means.

4. System according to any of the preceding claims, wherein the mutual spacing of the poles seen in the longitudinal direction of the channel is defined by a first angle (47') to the channel being lower than 89 degrees, preferably between 0 and 60 degrees, more preferably between 0 and 40 degrees, even more preferably between 0 and 20 degrees, or most preferably about 0 degrees

5. System according to any of the preceding claims for quantitative detection of the presence or absence of a target analyte in a liquid sample having a volume of less than $200\mu l$, wherein the paramagnetic particles in the channel of the channel device are comprised in an immobilisation matrix capable of capturing the analyte.

6. System according to claim 5, wherein the channel device further comprises:

   a) a first part comprising the first part of the capillary channel (3) and the sample inlet (1) at the first end (21) for the introduction of a sample containing an analyte;
   b) a second part (5, 6) comprising a washing zone (5) and a detection zone (6), said detection zone comprising a detection window (14) through which the target analyte can be detected by detection means;
   c) a solution inlet (8, 25, 26) at the second end for introduction of washing solutions and reaction mixtures; and
   d) a discharge outlet (4b, 27) for the discharge of waste products.

7. Use of the system according to any of the claims 5-6 for the quantitative detection of the presence or absence of a target analyte in a sample.

8. Method for clustering and/or homogeneously distributing a plurality of paramagnetic particles contained in a liquid in a longitudinal capillary channel (3, 5, 6) of a channel device (38), said channel having an inner surface and a first end (21) connected to a sample inlet (1) and a second end, said method comprising:

   a) solution of a layer comprising the plurality of paramagnetic particles with the liquid through the first end of the channel;
   b) applying a magnetic field to at least a portion of said channel by way of a magnetic field generating means (43) having first and second opposite poles, said magnetic field generating means being arranged relatively to the channel so that the poles thereof are mutually spaced viewed in the longitudinal direction of the channel;
   c) moving the magnetic field generating means longitudinally in relation to the channel;
   d) optional repeating step c) where the magnetic field is moved in the reverse longitudinal direction; and
   e) removing the magnetic field.

9. Method according to claim 8, wherein the mutual spacing of the poles seen in the longitudinal direction of the channel is defined by a first angle (47') to the channel being lower than 89 degrees, preferably between 0 and 60 degrees, more preferably between 0 and 40 degrees, even more preferably between 0 and 20 degrees, or most preferably about 0 degrees.

10. Method according to any of the claims 8-9, wherein the paramagnetic particles are comprised in an immobilisation matrix.

11. Method for quantitative detection of the presence or absence of a target analyte in a sample consisting of less than 200 $\mu l$ liquid, comprising the steps of:

   a) providing an analyte containing liquid sample consisting of less than $200\mu l$ liquid;
   b) supplying the liquid analyte containing sample to a first end (21) of a capillary channel and thereby solution of a layer as defined in step a) of the method in any of the claims 8-10, the capillary channel comprising a first reaction part (3) and a second detection part (5, 6), the two parts being physically separated such that sample material cannot enter into contact with the second detection part prior to step j) below;
   c) contacting the sample with an immobilisation matrix as defined in claim 27 capable of capturing the analyte;
   d) binding of the analyte to the immobilisation matrix in the first reaction chamber (3) by way of step c)-d) according to any of the claims 8-10;
   e) optional repeating step d) one or more times;
   f) immobilising the immobilisation matrix comprising the captured analyte as defined in step b) of the method according to any of the claims 8-10;

g) discarding the supernatant;

h) supplying a washing solution and washing the chamber as defined in step c)-d) of the method according to any of the claims 8-10, discarding the washing solution;

i) optional repeating step h) one or more times;

j) transferring the immobilisation matrix comprising the captured analyte to the second detection part (6) of the chamber as defined in step c) according to any of the claims 8-10; and

k) releasing the immobilisation matrix by way of step e) of the method according to any of the claims 8-10 and detecting the presence or absence of a target analyte by using conventional detection means.

12. Method according to claim 11 further comprising a step b') or c') of contacting the analyte with a signal generating biological marker capable of binding to the analyte.

13. Method according to claim 12, where the signal generating biological marker comprising an antibody.

14. Method according to any of the claims 12 or 13, where the signal generating biological marker comprising HRP (Horseradish Peroxidase) or ALP (Alkaline Phosphatase) conjugated to the biological marker.

15. Method according to any of the claims 12-14, where the step b') is performed after step b) and prior to step c).

16. Method according to any of the claims 12-14, where the step c') is performed after step c) and prior to step d).

17. Method according to any of the claims 11-16, where the immobilisation matrix comprises an antibody capable of binding the analyte.

Fig. 1

Fig. 2

Fig. 3

Fig. 4A

Fig. 4B

Fig. 5

Fig. 6

Fig. 7

Fig. 8

Fig. 9

Fig. 10

| | Europäisches Patentamt |
|---|---|
| | European Patent Office |
| | Office européen des brevets |

# EUROPEAN SEARCH REPORT

**Application Number**

EP 08 17 3113

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/110779 A (INVERNESS MEDICAL SWITZERLAND [CH]) 4 October 2007 (2007-10-04) * page 2, lines 10-27 * * page 3, line 32 - page 4, line 6 * * page 10, lines 6-14 * * page 11, lines 3-10 * * page 18, line 7 - line 19 * * page 61, line 30 - page 62, line 16 * * page 72, lines 10-19 * * page 110, lines 12-20 * * page 119, line 26 - page 120, line 5 * * page 120, lines 6-28 * * figures 1,3,5,48A-48C * ----- | 1-17 | INV. B01L3/00 G01N33/543 G01N35/00 G01N1/40 |
| X | EP 1 707 965 A (JAPAN SCIENCE & TECH AGENCY [JP]) 4 October 2006 (2006-10-04) * paragraphs [0015], [0022], [0024], [0025], [0033], [0064], [0077] * ----- | 1-4 | |
| X | US 2008/160639 A1 (SU XINA [US] ET AL) 3 July 2008 (2008-07-03) * paragraphs [0018], [0020], [0022], [0025], [0026], [0029], [0033], [0051], [0075] * ----- | 1-4 | TECHNICAL FIELDS SEARCHED (IPC) B01L G01N |

The present search report has been drawn up for all claims

| Place of search | Date of completion of the search | Examiner |
|---|---|---|
| Munich | 29 May 2009 | Hoyal, Barnaby |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 08 17 3113

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2009

| Patent document cited in search report | | Publication date | Patent family member(s) | | Publication date |
|---|---|---|---|---|---|
| WO 2007110779 | A | 04-10-2007 | AU | 2007231030 A1 | 04-10-2007 |
| | | | CA | 2645622 A1 | 04-10-2007 |
| | | | EP | 2010321 A2 | 07-01-2009 |
| | | | GB | 2446309 A | 06-08-2008 |
| EP 1707965 | A | 04-10-2006 | WO | 2005069015 A1 | 28-07-2005 |
| | | | KR | 20070037432 A | 04-04-2007 |
| | | | US | 2008226500 A1 | 18-09-2008 |
| US 2008160639 | A1 | 03-07-2008 | NONE | | |

EPO FORM P0459

For more details about this annex : see Official Journal of the European Patent Office, No. 12/82

**EP 2 208 531 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008116543 A
- WO 03061835 A
- WO 2004078316 A
- WO 2008010111 A
- EP 19970116857 A
- WO 03086637 A

### Non-patent literature cited in the description

- **Zborowski et al.** *Journal of Magnetism and Magnetic Materials,* vol. 194 (1-3), 224-230
- **Hayes, M.A.** Active control of dynamic supraparticle structures in microchannels. *Langmuir,* 2001, vol. 17 (9), 2866-2871
- **Rida, A.** Dynamics of magnetically retained supraparticles structures in liquid flow. *Applied Physics Letters,* 2004, vol. 85, 4986-4988